(19) Europäisches Patentamt
European Patent Office
Office européen des brevets

(11) **EP 2 414 053 B1**

(12) **FASCICULE DE BREVET EUROPEEN**

(45) Date de publication et mention
de la délivrance du brevet:
**27.11.2013 Bulletin 2013/48**

(21) Numéro de dépôt: **10712055.2**

(22) Date de dépôt: **29.03.2010**

(51) Int Cl.:
***A63B 69/00*** *(2006.01)*      ***A63B 71/06*** *(2006.01)*

(86) Numéro de dépôt international:
**PCT/EP2010/054130**

(87) Numéro de publication internationale:
**WO 2010/112469 (07.10.2010 Gazette 2010/40)**

(54) **SYSTÈME ET PROCÉDÉ D' OBSERVATION D'UNE ACTIVITÉ DE MARCHE D'UNE PERSONNE**

SYSTEM UND VERFAHREN ZUR BEOBACHTUNG DER GEHAKTIVITÄT EINER PERSON

SYSTEM AND METHOD FOR OBSERVING A PERSON'S WALKING ACTIVITY

(84) Etats contractants désignés:
**AT BE BG CH CY CZ DE DK EE ES FI FR GB GR
HR HU IE IS IT LI LT LU LV MC MK MT NL NO PL
PT RO SE SI SK SM TR**

(30) Priorité: **31.03.2009 FR 0952012**

(43) Date de publication de la demande:
**08.02.2012 Bulletin 2012/06**

(73) Titulaires:
• **Movea
38000 Grenoble (FR)**
• **Commissariat à l'Énergie Atomique
et aux Énergies Alternatives
75015 Paris (FR)**

(72) Inventeur: **JALLON, Pierre
F-38100 Grenoble (FR)**

(74) Mandataire: **Brunelli, Gérald
Marks & Clerk France
Immeuble Visium
22, avenue Aristide Briand
94117 Arcueil Cedex (FR)**

(56) Documents cités:
**EP-A1- 1 547 567     EP-A1- 2 011 552**

## Description

La présente invention porte sur un système et un procédé d'observation d'une activité de marche d'une personne, ou, en d'autres termes d'observation d'un déplacement d'une personne par un mode de locomotion constitué par une suite de pas.

Des systèmes d'analyse de mouvement de personnes sont de plus en plus répandus dans le domaine biomédical, notamment pour analyser l'activité physique d'une personne, voir par example le document EP-A-1547567.

La détection de l'activité de marche d'une personne est une information qui permet, par exemple, d'estimer une dépense énergétique d'une personne, d'évaluer un niveau de sédentarité d'une personne, ou d'estimer la qualité ou la perte de capacité fonctionnelle après une intervention chirurgicale ou un traitement médicamenteux.

Le document "Stair climbing detection during daily physical activity using a miniature gyroscope" de Brian Coley, Bijan Najafi, Anisoara Paraschiv_Ionescu, et Kamiar Aminian, paru dans Gait and Posture en 2005, porte sur la détection de la montée et de la descente d'escalier à l'aide d'un gyroscope. Pour cela, le système estime les différents instants caractéristiques d'un cycle de marche.

La présente invention vise à permettre une analyse qualitative d'une activité de marche d'une personne, ce qui peut être particulièrement utile pour surveiller précisément l'activité d'une personne.

Aussi, il est proposé, selon un aspect de l'invention, un système d'observation d'une activité de marche d'une personne, comprenant un dispositif adapté pour délivrer en sortie, pour un appui de pied de la personne, une première différence de vitesses angulaires du tibia correspondant entre l'instant auquel le talon du pied est posé et l'instant auquel le pied est à plat, une deuxième différence de vitesses angulaires du tibia correspondant entre l'instant auquel le talon du pied est posé et l'instant auquel le dernier orteil du pied est levé, et une vitesse angulaire du tibia correspondant à l'instant auquel le pied est à plat. Le système comprend, en outre, des moyens d'analyse des signaux délivrés par le dispositif adaptés pour déterminer un type de marche de l'utilisateur en fonction du temps en utilisant un modèle de Markov caché à N états correspondant respectivement à N types de marches.

Un tel système permet, à coût réduit, d'observer et de déterminer avec précision l'activité de marche d'une personne.

Selon un mode de réalisation, la densité de probabilité $p_x(\underline{x}(n))$ de correspondance entre les signaux délivrés par le dispositif et un état du modèle de Markov caché représentant un type de marche est définie par la première expression suivante :

$$\frac{1}{\sqrt{2\pi|\Sigma|}} \cdot e^{\frac{(\underline{x}(n)-\underline{\mu})^T \Sigma^{-1}(\underline{x}(n)-\underline{\mu})}{2}}$$

dans laquelle :

$\underline{x}(n)$     représente le vecteur colonne de composantes les trois signaux transmis par le dispositif ;

$\underline{\mu}$     représente un vecteur colonne à trois composantes, représentatif de l'état du modèle de Markov caché correspondant audit type de marche ; et

$|\Sigma|$     représente la valeur absolue du déterminant d'une matrice diagonale E de dimension 3 représentative de l'état du modèle de Markov caché correspondant audit type de marche.

L'utilisation d'un tel modèle de Markov caché, permet de distinguer précisément le type de marche pratiqué en temps réel.

Dans un mode de réalisation, la densité de probabilité de correspondance entre les signaux délivrés par le dispositif et un état du modèle de Markov caché représentant un type de marche est défini par une combinaison linéaire desdites premières expressions des densités de probabilités de l'ensemble des types de marche, la somme des coefficients de ladite combinaison linéaire valant 1.

Cela permet d'améliorer sensiblement la précision des résultats.

Selon un mode de réalisation, les moyens d'analyse sont adaptés pour déterminer le type de marche de l'utilisateur parmi un ensemble d'au moins deux types de marche parmi la marche à plat, la marche en descente, la marche en montée, la montée d'escalier, la descente d'escalier.

Ainsi, l'invention permet de distinguer tout type de marche parmi ces types de marche classiques.

Dans un mode de réalisation, pour la marche à plat, les trois composantes $\mu_1, \mu_2, \mu_3$ du vecteur colonne $\underline{\mu}$ sont telles que $\mu_1 \in [-1.2;-1]$, $\mu_2 \in [0.25;0.35]$, et $\mu_3 \in [-0.3;-0.2]$, et les trois composantes diagonales $\Sigma_1, \Sigma_2, \Sigma_3$ de la matrice diagonale $\Sigma$ sont telles que $\Sigma_1 \in [10^{-3};510^{-2}]$, $\Sigma_2 \in [10^{-4};510^{-3}]$, et $\Sigma_3 \in [10^{-4};510^{-2}]$.

Selon un mode de réalisation, pour la marche en descente, les trois composantes $\mu_1, \mu_2, \mu_3$ du vecteur colonne $\underline{\mu}$ sont telles que $\mu_1 \in [-1.4;-1]$, $\mu_2 \in [0.05;025]$, et $\mu_3 \in [-0.7;-0.2]$, et les trois composantes diagonales $\Sigma_1, \Sigma_2, \Sigma_3$ de la matrice diagonale E sont telles que $\Sigma_1 \in [10^{-3};510^{-2}]$, $\Sigma_2 \in [10^{-3};10^{-2}]$, et $\Sigma_3 \in [10^{-4};510^{-2}]$.

Dans un mode de réalisation, pour la marche en montée, les trois composantes $\mu_1, \mu_2, \mu_3$ du vecteur colonne $\underline{\mu}$ sont telles que $\mu_1 \in [-1.1;-0.2]$, $\mu_2 \in [0.1;0.2]$, et $\mu_3 \in [-1.2;-0.6]$, et les trois composantes diagonales $\Sigma_1, \Sigma_2, \Sigma_3$ de la matrice diagonale $\Sigma$ sont telles que $\Sigma_1 \in [10^{-3};510^{-2}]$, $\Sigma_2 \in [10^{-3};10^{-2}]$, et $\Sigma_3 \in [10^{-3};510^{-2}]$.

Selon un mode de réalisation, pour la montée d'escalier, les trois composantes $\mu_1, \mu_2, \mu_3$ du vecteur colonne $\underline{\mu}$ sont telles que $\mu_1 \in [-0.4;-0.15]$, $\mu_2 \in [0;0.2]$, et $\mu_3 \in [-0.7;-0.4]$, et les trois composantes diagonales $\Sigma_1, \Sigma_2, \Sigma_3$ de la matrice diagonale $\Sigma$ sont telles que $\Sigma_1 \in [10^{-2};10^{-1}]$, $\Sigma_2 \in [510^{-3};510^{-2}]$, et $\Sigma_3 \in [10^{-2};10^{-1}]$.

Dans un mode de réalisation, pour la descente d'escalier, les trois composantes $\mu_1, \mu_2, \mu_3$ du vecteur colonne $\underline{\mu}$ sont telles que $\mu_1 \in [-0.8;-0.6]$, $\mu_2 \in [-0.3;-0.1]$, et $\mu_3 \in [-0.4;-0.25]$, et les trois composantes diagonales $\Sigma_1, \Sigma_2, \Sigma_3$ de la matrice diagonale $\Sigma$ sont telles que $\Sigma_1 \in [10^{-3};10^{-2}]$, $\Sigma_2 \in [10^{-4};10^{-3}]$, et $\Sigma_3 \in [10^{-4};10^{-3}]$.

Selon un mode de réalisation, les probabilités P, dudit modèle de Markov caché, de passage entre deux états représentant respectivement un desdits type de marche sont telles que :

$P(état_i, état_j) \in [0.5;0.9999]$, lorsque i est différent de j; et
$P(état_i, état_j) \in [0;0.5]$, lorsque i est égal à j.

La précision du système est ainsi améliorée.

Dans un mode de réalisation, lesdits moyens d'analyse sont liés ou distants par rapport au dispositif, et le dispositif comprend des moyens de transmission avec ou sans fil pour transmettre ses mesures auxdits moyens d'analyse.

Ainsi, de nombreux modes de réalisation peuvent être envisagés.

Selon un mode de réalisation, le système comprend, en outre, des moyens d'affichage liés au dispositif et/ou des moyens d'affichages distants.

Ainsi, les résultats d'analyse de l'activité de marche de l'utilisateur peuvent être visualisés directement, ou bien analysés et visualisés sur un plus grand écran externe, par exemple d'un ordinateur portable qui peut comprendre les moyens d'analyse.

Selon un mode de réalisation, le dispositif comprend un magnétomètre, et/ou un accéléromètre, et/ou un gyromètre pour délivrer ses signaux de sortie.

Selon un autre aspect de l'invention, il est également proposé un procédé d'observation d'une activité de marche d'une personne, à partir, lors d'un appui de pied, de signaux représentant une première différence de vitesses angulaires du tibia correspondant entre l'instant auquel le talon du pied est posé et l'instant auquel le pied est à plat, une deuxième différence de vitesses angulaires du tibia correspondant entre l'instant auquel le talon du pied est posé et l'instant auquel le dernier orteil du pied est levé, et une vitesse angulaire du tibia correspondant à l'instant auquel le pied est à plat. On analyse lesdits signaux pour déterminer un type de marche de l'utilisateur en fonction du temps en utilisant un modèle de Markov caché à N états correspondant respectivement à N types de marches.

L'invention sera mieux comprise à l'étude de quelques modes de réalisation décrits à titre d'exemples nullement limitatifs et illustrés par les dessins annexés sur lesquels :

- les figures 1 et 2 illustrent deux modes de réalisation de systèmes selon un aspect de l'invention ; et
- la figure 3 illustre deux exemples de résultat d'un système selon un aspect de l'invention.

Dans l'ensemble de figures, les éléments ayants les mêmes références sont similaires.

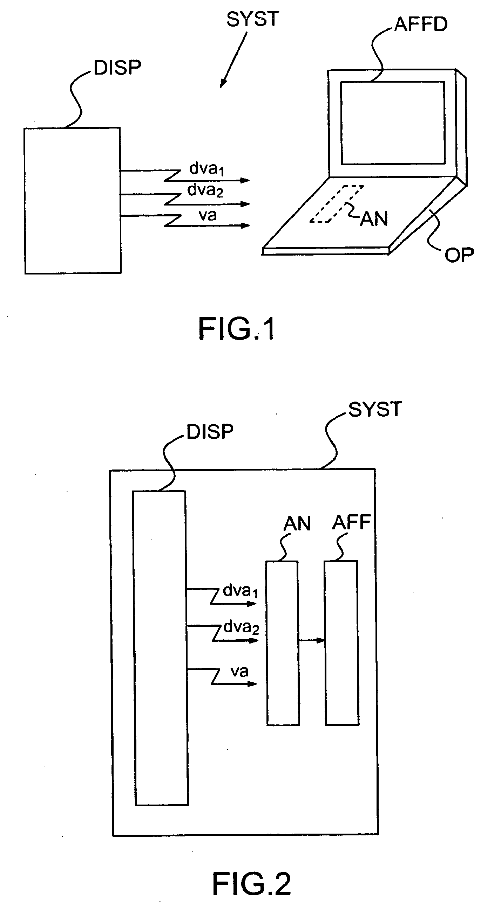

Tel qu'illustré sur la figure 1, le système SYST d'observation d'une activité de marche d'une personne comprend un dispositif DISP adapté pour délivrer en sortie, pour un appui de pied de la personne, une première différence $dva_1$ de vitesses angulaires du tibia correspondant entre l'instant auquel le talon du pied est posé et l'instant auquel le pied est à plat, une deuxième différence $dva_2$ de vitesses angulaires du tibia correspondant entre l'instant auquel le talon du pied est posé et l'instant auquel le dernier orteil du pied est levé, et une vitesse angulaire va du tibia correspondant à l'instant auquel le pied est à plat.

Un tel dispositif DISP est par exemple décrit dans la demande de brevet français FR 08/00571 comprenant un magnétomètre destiné à être fixé à un segment tibial de la personne pour générer un signal représentatif d'au moins une projection dans un plan sagittal d'un champ magnétique ambiant dans lequel il est immergé, et un module de traitement du signal pour identifier des instants et/ou des phases caractéristiques de la marche de la personne à partir du signal générer par le magnétomètre. En supposant que la personne se déplace dans un plan, c'est-à-dire que la personne marche selon une droite, il est possible à partir du magnétomètre d'estimer la vitesse angulaire du tibia. Cette information peut également être directement obtenu à partir d'un gyromètre. A partir de cette information, les instants caractéristiques du cycle de marche peuvent être déduits comme décrits dans le document "Stair climbing detection during daily physical activity using a miniature gyroscope" de Brian Coley, Bijan Najafi, Anisoara Paraschiv_Ionescu, et Kamiar Aminian.

En variante, le dispositif DISP peut comprendre un accéléromètre et/ou un gyromètre, et/ou un magnétomètre pour générer ses signaux de sortie.

Les signaux de sortie du dispositif DISP sont transmis, par exemple par liaison sans fil, à un module d'analyse AN, distants, par exemple intégré à un ordinateur portable OP muni d'un grand écran d'affichage AFFD.

En variante, comme illustré sur la figure 2, le module d'analyse peut être lié au dispositif DISP, et ce dernier peut être muni d'un écran d'affichage AFF.

Dans la suite de la description, l'exemple nullement limitatif traité, est un système selon un aspect de l'invention, dans lequel le module d'analyse AN est adapté pour déterminer le type de marche de l'utilisateur en fonction du temps en utilisant un modèle de Markov caché à 5 états (N=5) correspondant respectivement à la marche à plat (état 1), la marche en descente (état 2), la marche en montée (état 3), la montée d'escalier (état 4), et la descente d'escalier (état 5).

Ces trois signaux fournis par le dispositif DISP sont notés sous forme d'un vecteur à trois dimensions $\underline{x}(n)=[x_1(n),x_2,(n),x_3(n)]^T =[dva_1(n),dva_2(n),va(n)]^T$, dans lequel n représente l'indice de l'échantillon à la fréquence fournie par le dispositif DISP, et x1, x2, et x3 représentent les valeurs des signaux correspondant aux trois signaux délivrés par le dispositif DISP, i.e. dva1, dva2, et va.

La densité de probabilité $p_x (\underline{x}(n))$ de correspondance entre les signaux délivrés par le dispositif et un état du modèle de Markov caché représentant un type de marche est définie par la première expression suivante :

$$\frac{1}{\sqrt{2\pi|\Sigma|}} \cdot e^{\frac{(\underline{x}(n)-\underline{\mu})^T \Sigma^{-1} (\underline{x}(n)-\underline{\mu})}{2}}$$

dans laquelle :

$\underline{x}(n)$     représente le vecteur colonne de composantes les trois signaux transmis par le dispositif DISP ;

$\underline{\mu}$     représente un vecteur colonne à trois composantes, représentatif de l'état du modèle de Markov caché correspondant audit type de marche ; et

$|\Sigma|$     représente la valeur absolue du déterminant d'une, matrice diagonale $\Sigma$ de dimension 3 représentative de l'état du modèle de Markov caché correspondant audit type de marche.

Les cinq types de marche de l'exemple considéré sont définis par les paramètres suivants correspondant à la première expression de la densité de probabilité $p_{x,k}$ associé à l'état k :

- la marche à plat (état 1 du modèle de Markov caché) :

$$\underline{\mu} = [1.4421; -1.8292; -0.9000]^T \text{ et } \Sigma = \begin{bmatrix} 0.145 & 0 & 0 \\ 0 & 0.40 & 0 \\ 0 & 0 & 0.05 \end{bmatrix}$$

- la marche en descente (état 2 du modèle de Markov caché) :

$$\underline{\mu} = [1.9393; -1.0854; -0.6676]^T \text{ et } \Sigma = \begin{bmatrix} 0.041 & 0 & 0 \\ 0 & 0.073 & 0 \\ 0 & 0 & 0.1 \end{bmatrix}$$

- la marche en montée (état 3 du modèle de Markov caché) :

$$\underline{\mu} = [2.88; 0.24; -0.34]^T \text{ et } \Sigma = \begin{bmatrix} 0.95 & 0 & 0 \\ 0 & 0.24 & 0 \\ 0 & 0 & 0.45 \end{bmatrix}$$

- la montée d'escalier (état 4 du modèle de Markov caché) :

$$\underline{\mu} = [1.14; -2.19; 0.46]^T \ \text{ et } \ \Sigma = \begin{bmatrix} 0.39 & 0 & 0 \\ 0 & 0.60 & 0 \\ 0 & 0 & 0.16 \end{bmatrix}$$

- la descente d'escalier (état 5 du modèle de Markov caché) :

$$\underline{\mu} = [0.70; -1.0; -0.35]^T \ \text{ et } \ \Sigma = \begin{bmatrix} 0.15 & 0 & 0 \\ 0 & 0.10 & 0 \\ 0 & 0 & 0.05 \end{bmatrix}$$

Les densités de probabilités de passage P(état$_i$ / état$_j$) d'un état état$_i$ correspondant à un type de marche du modèle de Markov caché à un autre état état$_j$ correspondant à un type de marche du modèle de Markov caché sont les suivantes, choisies de manière à assurer une bonne stabilité au système :

| P(état$_j$ / état$_i$) | état$_j$=1 (marche à plat) | état$_j$=2 (marche en descente) | état$_j$=3 (marche en montée) | état$_j$=4 (montée d'escalier) | état$_j$=5 (descente d'escalier) |
|---|---|---|---|---|---|
| état$_i$=1 (marche à plat) | 0.99 | 0.0025 | 0.0025 | 0.0025 | 0.0025 |
| état$_i$=2 (marche en descente) | 0.0025 | 0.99 | 0.0025 | 0.0025 | 0.0025 |
| état$_i$=3 (marche en montée) | 0.0025 | 0.0025 | 0.99 | 0.0025 | 0.0025 |
| état$_i$=4 (montée d'escalier) | 0.0025 | 0.0025 | 0.0025 | 0.99 | 0.0025 |
| état$_i$=5 (descente d'escalier) | 0.0025 | 0.0025 | 0.0025 | 0.0025 | 0.99 |

Un tel système permet de distinguer les passages entre les différents types de marche d'un utilisateur. Les probabilités de passage sont choisies suffisamment proches de 1 pour assurer une bonne stabilité au système. En effet, il est nécessaire d'observer durant au moins un certain délai que la nature des signaux a changée pour que le module d'analyse AN décide que le type de marche a changé.

Il est possible d'améliorer sensiblement les performances du système en définissant la densité de probabilité de correspondance entre les signaux délivrés par le dispositif DISP et un état du modèle de Markov caché représentant un type de marche par une combinaison linéaire desdites premières expressions des densités de probabilités de l'ensemble des types de marche, la somme des coefficients de ladite combinaison linéaire valant 1.

On pose ainsi, pour l'état i du modèle de Markov caché :

$$P_X^i \left( \underline{x}(n) \right) = \sum_{k=1}^{5} \alpha_{i,k} \, p_{X,k} \left( \underline{x}(n) \right)$$

$p_{x,k} \left( \underline{x}(n) \right)$     étant ladite première expression de la densité de probabilité $p_{x,k}$ associé à l'état k.

$\alpha_{i,k}$                étant un coefficient de pondération

avec $\sum_{k=1}^{5} \alpha_{i,k} = 1$

Dans cet exemple, les valeurs suivantes ont été utilisées pour les coefficients de pondération :

|  | k=1 (marche à plat) | k=2 (marche en descente) | k=3 (marche en montée) | k=4 (montée d'escalier) | k=5 (descente d'escalier) |
|---|---|---|---|---|---|
| $\alpha_{1,k}$ | 0.9 | 0 | 0 | 0 | 0.1 |
| $\alpha_{2,k}$ | 0.025 | 0.9 | 0.025 | 0.025 | 0.0025 |
| $\alpha_{3,k}$ | 0.025 | 0.025 | 0.9 | 0.025 | 0.0025 |
| $\alpha_{4,k}$ | 0.025 | 0.025 | 0.025 | 0.9 | 0.0025 |
| $\alpha_{5,k}$ | 0 | 0 | 0 | 0.03 | 0.97 |

Le module d'analyse AN détermine, à partir des signaux d'entrée et du modèle de Markov caché tel que défini, la séquence d'états (types de marche) la plus probable, selon des procédés classiques, par exemple en calculant pour l'ensemble des séquences d'états possibles la probabilité associée compte tenu du signal observé et en gardant la séquence la plus probable, tels que décrits par exemple dans le document "An introduction to hidden Markov models" de L.R. Rabiner et B.H. Juang, IEEE ASSP Magazine, January 1986, ou dans le livre "Inference in Hidden Markov Models" de Cappé, Moulines et Ryden de Springer, de la série "Springer series in statisctics".

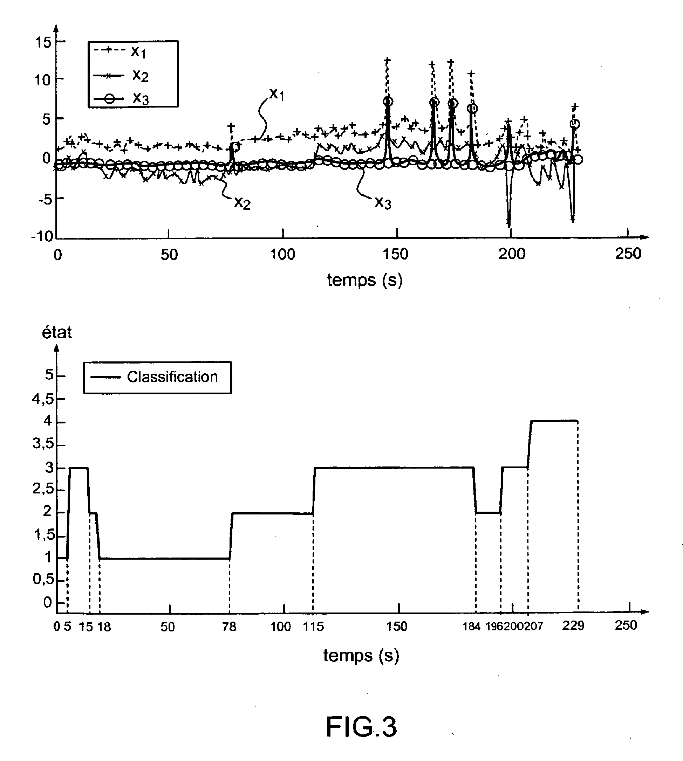

La figure 3 illustre un exemple d'enregistrement d'une séance de marche d'un utilisateur du système, avec sur le premier graphique les trois courbes représentant les valeurs de $x_1$, $x_2$ et $x_3$ valant respectivement $dva_1$, $dva_2$ et $va$ transmises par le dispositif DISP, en fonction du temps, et sur le deuxième graphique, l'état de marche du patient déterminé par le système SYST.

Sur cet exemple, l'utilisateur a marché à plat (état 1) pendant 5 secondes, puis a marché en montée (état 3) pendant 10 secondes, puis a marché en descente (état 2) pendant 3 secondes, puis a marché à plat (état 1) pendant 60 secondes, puis a marché en descente (état 2) pendant 37 secondes, puis a marché en montée (état 3) pendant 69 secondes, puis a marché en descente (état 2) pendant 12 secondes, puis a marché en montée (état 3) pendant 11 secondes, puis a monté un escalier (état 4) pendant 22 secondes.

La présente invention, permet, à coût réduit, de déterminer l'activité de marche d'une personne avec une précision améliorée.

**Revendications**

1. Système d'observation d'une activité de marche d'une personne, comprenant un dispositif (DISP) adapté pour délivrer en sortie, pour un appui de pied de la personne, une première différence de vitesses angulaires du tibia correspondant entre l'instant auquel le talon du pied est posé et l'instant auquel le pied est à plat, une deuxième différence de vitesses angulaires du tibia correspondant entre l'instant auquel le talon du pied est posé et l'instant auquel le dernier orteil du pied est levé, et une vitesse angulaire du tibia correspondant à l'instant auquel le pied est à plat, **caractérisé en ce qu'**il comprend des moyens d'analyse (AN) des signaux délivrés par le dispositif adaptés pour déterminer un type de marche de l'utilisateur en fonction du temps en utilisant un modèle de Markov caché à N états correspondant respectivement à N types de marches.

2. Système selon l'une des revendications précédentes, dans lequel la densité de probabilité $p_\chi(\underline{x}(n))$ de correspondance entre les signaux délivrés par le dispositif et un état du modèle de Markov caché représentant un type de marche est définie par la première expression suivante :

$$\frac{1}{\sqrt{2\pi|\Sigma|}} \cdot e^{\frac{\left(\underline{x}(n)-\underline{\mu}\right)^T \Sigma^{-1}\left(\underline{x}(n)-\underline{\mu}\right)}{2}}$$

dans laquelle :

$\underline{x}(n)$ représente le vecteur colonne de composantes les trois signaux transmis par le dispositif ;

$\underline{\mu}$ représente un vecteur colonne à trois composantes, représentatif de l'état du modèle de Markov caché correspondant audit type de marche ; et

$|\Sigma|$ représente la valeur absolue du déterminant d'une matrice diagonale $\Sigma$ de dimension 3 représentative de l'état du modèle de Markov caché correspondant audit type de marche.

3. Système selon la revendication 2 , dans lequel la densité de probabilité de correspondance entre les signaux délivrés par le dispositif (DISP) et un état du modèle de Markov caché représentant un type de marche est définie par une combinaison linéaire desdites premières expressions des densités de probabilités de l'ensemble des types de marche, la somme des coefficients de ladite combinaison linéaire valant 1.

4. Système selon l'une des revendications précédentes, dans lequel les moyens d'analyse (AN) sont adaptés pour déterminer le type de marche de l'utilisateur parmi un ensemble d'au moins deux types de marche parmi la marche à plat, la marche en descente, la marche en montée, la montée d'escalier, et la descente d'escalier.

5. Système selon la revendication 4, dans lequel, pour la marche à plat, les trois composantes $\mu_1, \mu_2, \mu_3$ du vecteur colonne $\underline{\mu}$ sont telles que $\mu_1 \in$ [-1.2;-1], $\mu_2 \in$ [0.25;0.35], et $\mu_3 \in$ [-0.3;-0.2], et les trois composantes diagonales $\Sigma_1, \Sigma_2, \Sigma_3$ de la matrice diagonale $\Sigma$ sont telles que $\Sigma_1 \in$ [$10^{-3}$;$510^{-2}$], $\Sigma_2 \in$ [$10^{-4}$;$510^{-3}$], et $\Sigma_3 \in$ [$10^{-4}$;$510^{-2}$].

6. Système selon la revendication 4 ou 5, dans lequel, pour la marche en descente, les trois composantes $\mu_1, \mu_2, \mu_3$ du vecteur colonne $\underline{\mu}$ sont telles que $\mu_1 \in$ [-1.4;-1], $\mu_2 \in$ [0.05;0.25], et $\mu_3 \in$ [-0.7;-0.2], et les trois composantes diagonales $\Sigma_1, \Sigma_2, \Sigma_3$ de la matrice diagonale $\Sigma$ sont telles que $\Sigma_1 \in$ [$10^{-3}$;$510^{-2}$], $\Sigma_2 \in$ [$10^{-3}$;$10^{-2}$], et $\Sigma_3 \in$ [$10^{-4}$;$510^{-2}$].

7. Système selon la revendication 4, dans lequel, pour la marche en montée, les trois composantes $\mu_1, \mu_2, \mu_3$ du vecteur colonne $\underline{\mu}$ sont telles que $\mu_1 \in$ [-1.1;-0.2], $\mu_2 \in$ [0.1;0.2], et $\mu_3 \in$ [-1.2;-0.6], et les trois composantes diagonales $\Sigma_1, \Sigma_2, \Sigma_3$ de la matrice diagonale $\Sigma$ sont telles que $\Sigma_1 \in$ [$10^{-3}$;$510^{-2}$], $\Sigma_2 \in$ [$10^{-3}$;$10^{-2}$], et $\Sigma_3 \in$ [$10^{-3}$;$510^{-2}$].

8. Système selon la revendication 4, dans lequel, pour la montée d'escalier, les trois composantes $\mu_1, \mu_2, \mu_3$ du vecteur colonne $\underline{\mu}$ sont telles que $\mu_1, \in$ [-0.4;-0.15], $\mu_2 \in$ [0;0.2], et $\mu_3 \in$ [-0.7;-0.4], et les trois composantes diagonales $\Sigma_1, \Sigma_2, \Sigma_3$ de la matrice diagonale $\Sigma$ sont telles que $\Sigma_1 \in$ [$10^{-2}$;$10^{-1}$], $\Sigma_2 \in$ [$510^{-3}$;$510^{-2}$], et $\Sigma_3 \in$ [$10^{-2}$;$10^{-1}$].

9. Système selon la revendication 4, dans lequel, pour la descente d'escalier, les trois composantes $\mu_1, \mu_2, \mu_3$ du vecteur colonne $\underline{\mu}$ sont telles que $\mu_1 \in$ [-0.8;-0.6], $\mu_2 \in$ [-0.3;-0.1], et $\mu_3 \in$ [-0.4;-0.25], et les trois composantes diagonales $\Sigma_1, \Sigma_2, \Sigma_3$ de la matrice diagonale $\Sigma$ sont telles que $\Sigma_1 \in$ [$10^{-3}$;$10^{-2}$], $\Sigma_2 \in$ [$10^{-4}$;$10^{-3}$], et $\Sigma_3 \in$ [$10^{-4}$;$10^{-3}$].

10. Système selon l'une des revendications 3 à 9, dans lequel les probabilités P, dudit modèle de Markov caché, de passage entre deux états représentant respectivement un desdits type de marche sont telles que :

$P(état_i, état_j) \in$ [0.5;0.9999], lorsque i est différent de j; et

$P(état_i, état_j) \in$ [0;0.5], lorsque i est égal à j

11. Système selon l'une des revendications précédentes, dans lequel lesdits moyens d'analyse (AN) sont liés ou distants par rapport au dispositif (DISP), et le dispositif (DISP) comprend des moyens de transmission avec ou sans fil pour transmettre ses mesures auxdits moyens d'analyse (AN).

12. Système selon l'une des revendications précédentes, comprenant, en outre, des moyens d'affichage (AFF) liés au dispositif et/ou des moyens d'affichages distants (AFFD).

13. Système selon l'une des revendications précédentes, dans lequel ledit dispositif (DISP) comprend un magnétomètre, et/ou un accéléromètre, et/ou un gyromètre pour délivrer ses signaux de sortie.

14. Procédé d'observation d'une activité de marche d'une personne, à partir, lors d'un appui de pied, de signaux représentant une première différence de vitesses angulaires du tibia correspondant entre l'instant auquel le talon du pied est posé et l'instant auquel le pied est à plat, une deuxième différence de vitesses angulaires du tibia correspondant entre l'instant auquel le talon du pied est posé et l'instant auquel le dernier orteil du pied est levé, et une vitesse angulaire du tibia correspondant à l'instant auquel le pied est à plat, **caractérisé en ce que** l'on analyse (AN) lesdits signaux pour déterminer un type de marche de l'utilisateur en fonction du temps en utilisant un modèle de Markov caché à N états correspondant respectivement à N types de marches.

**Patentansprüche**

1.  System zum Beobachten der Gehaktivität einer Person, das eine Vorrichtung (DISP) umfasst, die so ausgelegt ist, dass sie als Ausgang für ein Auftreten mit dem Fuß der Person eine erste Winkelgeschwindigkeitsdifferenz des entsprechenden Schienbeins zwischen dem Zeitpunkt, an dem die Ferse des Fußes aufgesetzt wird, und dem Zeitpunkt liefert, an dem der Fuß eben ist, und eine zweite Winkelgeschwindigkeitsdifferenz des entsprechenden Schienbeins zwischen dem Zeitpunkt, an dem die Ferse des Fußes aufgesetzt wird, und dem Zeitpunkt liefert, an dem der letzte Zeh des Fußes angehoben ist, und eine Winkelgeschwindigkeit des entsprechenden Schienbeins zu dem Zeitpunkt liefert, an dem der Fuß eben ist, **dadurch gekennzeichnet, dass** es Mittel (AN) zum Analysieren von von der Vorrichtung gelieferten Signalen umfasst, wobei die Mittel so ausgelegt sind, dass sie eine Gangart des Benutzers in Abhängigkeit von der Zeit anhand eines versteckten Markov-Modells mit N Zuständen ermittelt, die jeweils N Gangarten entsprechen.

2.  System nach dem vorherigen Anspruch, wobei die Wahrscheinlichkeitsdichte $p_x(\underline{x}(n))$ der Korrespondenz zwischen den von der Vorrichtung gelieferten Signalen und einem Zustand des versteckten Markov-Modells, der eine Gangart repräsentiert, durch den folgenden ersten Ausdruck definiert wird:

$$\frac{1}{\sqrt{2\pi|\Sigma|}} \cdot e^{\frac{\left(\underline{x}(n)-\underline{\mu}\right)^T \Sigma^{-1}\left(\underline{x}(n)-\underline{\mu}\right)}{2}}$$

wobei:

$\underline{x}(n)$ den Spaltenvektor von Komponenten der drei Signale repräsentiert, die von der Vorrichtung übertragen werden;
$\underline{\mu}$ einen Spaltenvektor mit drei Komponenten repräsentiert, die den Zustand des versteckten Markov-Modells repräsentieren, der der Gangart entspricht; und
$|\Sigma|$ den Absolutwert der Determinanten einer diagonalen Matrix $\Sigma$ von 3 Dimensionen repräsentiert, die den Zustand des versteckten Markov-Modells repräsentieren, der der Gangart entspricht.

3.  System nach Anspruch 2, wobei die Wahrscheinlichkeitsdichte der Korrespondenz zwischen den von der Vorrichtung (DISP) gelieferten Signalen und einem Zustand des versteckten Markov-Modells, der eine Gangart repräsentiert, durch eine lineare Kombination der ersten Ausdrücke der Wahrscheinlichkeitsdichten aller Gangarten definiert wird, wobei die Summe der Koeffizienten der linearen Kombination gleich 1 ist.

4.  System nach einem der vorherigen Ansprüche, wobei die Analysemittel (AN) so ausgelegt sind, dass sie die Gangart des Benutzers aus einem Satz von wenigstens zwei Gangarten aus Gehen auf ebenem Boden, Bergabgehen, Bergaufgehen, Treppenaufstieg und Treppenabstieg ermitteln.

5.  System nach Anspruch 4, wobei zum Gehen auf ebenem Boden die drei Komponenten $\mu_1$, $\mu_2$, $\mu_3$ des Spaltenvektors $\underline{\mu}$ derart sind, dass $\mu_1 \in [-1,2;-1]$, $\mu_2 \in [0,25;0,35]$ und $\mu_3 \in [-0,3;-0,2]$ ist, und die drei diagonalen Komponenten $\Sigma_1$, $\Sigma_2$, $\Sigma_3$ der diagonalen Matrix $\Sigma$ derart sind, dass $\Sigma_1 \in [10^{-3};5 \times 10^{-2}]$, $\Sigma_2 \in [10^{-4};5 \times 10^{-3}]$ und $\Sigma_3 \in [10^{-4};5\times10^{-2}]$ ist.

6.  System nach Anspruch 4, wobei zum Bergabgehen die drei Komponenten $\mu_1$, $\mu_2$, $\mu_3$ des Spaltenvektors $\underline{\mu}$ derart sind, dass $\mu_1 \in [-1,4;-1]$, $\mu_2 \in [0,05;0,25]$ und $\mu_3 \in [-0,7;-0,2]$ ist, und die drei diagonalen Komponenten $\Sigma_1$, $\Sigma_2$, $\Sigma_3$ der diagonalen Matrix $\Sigma$ derart sind, dass $\Sigma_1 \in [10^{-3};5 \times 10^{-2}]$, $\Sigma_2 \in [10^{-3};10^{-2}]$ und $\Sigma_3 \in [10^{-4}; 5 \times 10^{-2}]$ ist.

7.  System nach Anspruch 4, wobei zum Bergaufgehen die drei Komponenten $\mu_1$, $\mu_2$, $\mu_3$ des Spaltenvektors $\underline{\mu}$ derart sind, dass $\mu_1 \in [-1,1;-0,2]$, $\mu_2 \in [0,1;0,2]$ und $\mu_3 \in [-1,2;-0,6]$ ist, und die drei diagonalen Komponenten $\Sigma_1$, $\Sigma_2$, $\Sigma_3$ der diagonalen Matrix $\Sigma$ derart sind, dass $\Sigma_1 \in [10^{-3};5 \times 10^{-2}]$, $\Sigma_2 \in [10^{-3};10^{-2}]$ und $\Sigma_3 \in [10^{-3};5 \times 10^{-2}]$ ist.

8.  System nach Anspruch 4, wobei zum Treppenaufsteigen die drei Komponenten $\mu_1$, $\mu_2$, $\mu_3$ des Spaltenvektors $\underline{\mu}$ derart sind, dass $\mu_1 \in [-0,4;-0,15]$, $\mu_2 \in [0;0,2]$ und $\mu_3 \in [-0,7;-0,4]$ ist, und die drei diagonalen Komponenten $\Sigma_1$, $\Sigma_2$, $\Sigma_3$ der diagonalen Matrix $\Sigma$ derart sind, dass $\Sigma_1 \in [10^{-2};10^{-1}]$, $\Sigma_2 \in [5 \times 10^{-3};5 \times 10^{-2}]$ und $\Sigma_3 \in [10^{-2};10^{-1}]$ ist.

9.  System nach Anspruch 4, wobei zum Treppenabsteigen die drei Komponenten $\mu_1$, $\mu_2$, $\mu_3$ des Spaltenvektors $\underline{\mu}$.

derart sind, dass $\mu_1 \in$ [-0,8;-0,6], $\mu_2 \in$ [-0,3;-0,1] und $\mu_3 \in$ [-0,4;-0,25] ist, und die drei diagonalen Komponenten $\Sigma_1$, $\Sigma_2$, $\Sigma_3$ der diagonalen Matrix $\Sigma$ derart sind, dass $\Sigma_1 \in$ [10$^{-3}$;10$^{-2}$], $\Sigma_2 \in$ [10$^{-4}$;10$^{-3}$] und $\Sigma_3 \in$ [10$^{-4}$;10$^{-3}$] ist.

10. System nach einem der Ansprüche 3 bis 9, wobei die Wahrscheinlichkeiten P des versteckten Markov-Modells zum Umschalten zwischen zwei Zuständen, die jeweils eine der Gangarten repräsentieren, derart sind, dass:

*P(Zustand$_i$, Zustand$_j$)* $\in$ [0,5;0,9999], wobei i sich von j unterscheidet; und
*P(Zustand$_i$, Zustand$_j$)* $\in$ [0;0,5], wobei i gleich j ist.

11. System nach einem der vorherigen Ansprüche, wobei die Analysemittel (AN) mit der Vorrichtung (DISP) verbunden oder fern von dieser sind, und die Vorrichtung (DISP) verdrahtete oder drahtlose Übertragungsmittel zum Übertragen ihrer Messwerte zu den Analysemitteln (AN) umfasst.

12. System nach einem der vorherigen Ansprüche, das ferner mit der Vorrichtung verbundene Anzeigemittel (AFF) und/oder Fernanzeigemittel (AFFD) umfasst.

13. System nach einem der vorherigen Ansprüche, wobei die Vorrichtung (DISP) ein Magnetometer und/oder einen Beschleunigungsmesser und/oder ein Gyrometer zum Liefern ihrer Ausgangssignale umfasst.

14. Verfahren zum Beobachten der Gehaktivität einer Person anhand von Signalen, beim Auftreten, die eine erste Winkelgeschwindigkeitsdifferenz des entsprechenden Schienbeins zwischen dem Zeitpunkt, an dem die Ferse des Fußes aufgesetzt wird, und dem Zeitpunkt repräsentieren, an dem der Fuß eben ist, eine zweite Winkelgeschwindigkeitsdifferenz des entsprechenden Schienbeins zwischen dem Zeitpunkt, an dem die Ferse des Fußes aufgesetzt wird, und dem Zeitpunkt repräsentieren, an dem der letzte Zeh des Fußes angehoben ist, und eine Winkelgeschwindigkeit des entsprechenden Schienbeins zu dem Zeitpunkt repräsentieren, an dem der Fuß eben ist, **dadurch gekennzeichnet, dass** die Signale analysiert (AN) werden, um eine Gangart des Benutzers in Abhängigkeit von der Zeit anhand eines versteckten Markov-Modells mit N Zuständen zu ermitteln, die jeweils N Gangarten entsprechen.

**Claims**

1. A system for observing the walking activity of a person, comprising a device (DISP) that is designed to deliver as output, for a footstep of said person, a first difference in angular speeds of the corresponding tibia between the instant at which the heel of the foot is posed and the instant at which the foot is flat, a second difference in angular speeds of the corresponding tibia between the instant at which the heel of the foot is posed and the instant at which the last toe of the foot is raised, and an angular speed of the corresponding tibia at the instant at which the foot is flat, **characterised in that** it comprises means (AN) for analysing signals delivered by said device, which means are designed to determine a walking style of the user as a function of time by using a hidden Markov model with N states that respectively correspond to N walking styles.

2. The system according to the preceding claim, wherein the probability density $p_x(\underline{x}(n))$ of correspondence between the signals that are delivered by said device and a state of said hidden Markov model that represents a walking style is defined by the following first expression:

$$\frac{1}{\sqrt{2\pi|\Sigma|}} \cdot e^{\frac{\left(\underline{x}(n)-\underline{\mu}\right)^T \Sigma^{-1}\left(\underline{x}(n)-\underline{\mu}\right)}{2}}$$

wherein:

$\underline{x}(n)$ represents the column vector of components of the three signals that are transmitted by said device;
$\underline{\mu}$ represents a column vector with three components that represent the state of said hidden Markov model that corresponds to said walking style; and
$|\Sigma|$ represents the absolute value of the determinant of a diagonal matrix $\Sigma$ of 3 dimensions that represent the state of said hidden Markov model that corresponds to said walking style.

3. The system according to claim 2, wherein the probability density of correspondence between the signals that are delivered by said device (DISP) and a state of said hidden Markov model that represents a walking style is defined by a linear combination of said first expressions of the densities of probabilities of all of the walking styles, the total of the coefficients of said linear combination being equal to 1.

4. The system according to any one of the preceding claims, wherein said analysis means (AN) are designed to determine the walking style of said user from a set of at least two walking styles from among walking on the flat, walking downhill, walking uphill, climbing a staircase and descending a staircase.

5. The system according to claim 4, wherein, for walking on the flat, the three components $\mu_1$, $\mu_2$, $\mu_3$ of the column vector $\underline{\mu}$ are such that $\mu_1 \in [-1.2;-1]$, $\mu_2 \in [0.25;0.35]$ and $\mu_3 \in [-0.3;-0.2]$, and the three diagonal components $\Sigma_1$, $\Sigma_2$, $\Sigma_3$ of the diagonal matrix $\Sigma$ are such that $\Sigma_1 \in [10^{-3};5 \times 10^{-2}]$, $\Sigma_2 \in [10^{-4};5 \times 10^{-3}]$ and $\Sigma_3 \in [10^{-4};5 \times 10^{-2}]$.

6. The system according to claim 4, wherein, for walking downhill, the three components $\mu_1$, $\mu_2$, $\mu_3$ of the column vector $\underline{\mu}$ are such that $\mu_1 \in [-1.4;-1]$, $\mu_2 \in [0.05;0.25]$ and $\mu_3 \in [-0.7;-0.2]$, and the three diagonal components $\Sigma_1$, $\Sigma_2$, $\Sigma_3$ of the diagonal matrix $\Sigma$ are such that $\Sigma_1 \in [10^{-3};5 \times 10^{-2}]$, $\Sigma_2 \in [10^{-3};10^{-2}]$ and $\Sigma_3 \in [10^{-4};5 \times 10^{-2}]$.

7. The system according to claim 4, wherein, for walking uphill, the three components $\mu_1$, $\mu_2$, $\mu_3$ of the column vector $\underline{\mu}$ are such that $\mu_1 \in [-1.1;-0.2]$, $\mu_2 \in [0.1;0.2]$ and $\mu_3 \in [-1.2;-0.6]$, and the three diagonal components $\Sigma_1$, $\Sigma_2$, $\Sigma_3$ of the diagonal matrix $\Sigma$ are such that $\Sigma_1 \in [10^{-3};5 \times 10^{-2}]$, $\Sigma_2 \in [10^{-3};10^{-2}]$ and $\Sigma_3 \in [10^{-3};5 \times 10^{-2}]$.

8. The system according to claim 4, wherein, for climbing a staircase, the three components $\mu_1$, $\mu_2$, $\mu_3$ of the column vector $\underline{\mu}$ are such that $\mu_1, \in [-0.4;-0.15]$, $\mu_2 \in [0;0.2]$ and $\mu_3 \in [-0.7;-0.4]$, and the three diagonal components $\Sigma_1$, $\Sigma_2$, $\Sigma_3$ of the diagonal matrix $\Sigma$ are such that $\Sigma_1 \in [10^{-2};10^{-1}]$, $\Sigma_2 \in [5 \times 10^{-3};5 \times 10^{-2}]$ and $\Sigma_3 \in [10^{-2};10^{-1}]$.

9. The system according to any one of claims 4, wherein, for descending a staircase, the three components $\mu_1$, $\mu_2$, $\mu_3$ of the column vector $\underline{\mu}$ are such that $\mu_1 \in [-0.8;-0.6]$, $\mu_2 \in [-0.3;-0.1]$ and $\mu_3 \in [-0.4;-0.25]$, and the three diagonal components $\Sigma_1$, $\Sigma_2$, $\Sigma_3$ of the diagonal matrix $\Sigma$ are such that $\Sigma_1 \in [10^{-3};10^{-2}]$, $\Sigma_2 \in [10^{-4};10^{-3}]$ and $\Sigma_3 \in [10^{-4};10^{-3}]$.

10. The system according to any one of claims 3 to 9, wherein the probabilities P of said hidden Markov model for switching between two states that respectively represent one of said walking styles are such that:

$P(state_i, state_j) \in [0.5;0.9999]$, where i is different from j; and
$P(state_i, state_j) \in [0;0.5]$, where i is equal to j.

11. The system according to any one of the preceding claims, wherein said analysis means (AN) are connected to or are remote from said device (DISP) and said device (DISP) comprises wired or wireless transmission means for transmitting its measurements to said analysis means (AN).

12. The system according to any one of the preceding claims, further comprising display means (AFF) that are connected to said device and/or remote display means (AFFD).

13. The system according to any one of the preceding claims, wherein said device (DISP) comprises a magnetometer and/or an accelerometer and/or a gyrometer for delivering its output signals.

14. A method for observing the walking activity of a person, on the basis of signals, during a footstep, that represent a first difference in angular speeds of the corresponding tibia between the instant at which the heel of the foot is posed and the instant at which the foot is flat, a second difference in angular speeds of the corresponding tibia between the instant at which the heel of the foot is posed and the instant at which the last toe of the foot is raised, and an angular speed of the corresponding tibia at the instant at which the foot is flat, **characterised in that** said signals are analysed (AN) so as to determine a walking style of the user as a function of time by using a hidden Markov model with N states that respectively correspond to N walking styles.

SYST

DISP

AFFD

dva₁
dva₂
va

AN

OP

## FIG.1

DISP

SYST

AN   AFF

dva₁

dva₂

va

## FIG.2

FIG.3

## RÉFÉRENCES CITÉES DANS LA DESCRIPTION

**Documents brevets cités dans la description**

- EP 1547567 A

- FR 0800571

**Littérature non-brevet citée dans la description**

- **BRIAN COLEY ; BIJAN NAJAFI ; ANISOARA PARASCHIV_IONESCU ; KAMIAR AMINIAN.** Stair climbing detection during daily physical activity using a miniature gyroscope. *Gait and Posture,* 2005

- **L.R. RABINER ; B.H. JUANG.** An introduction to hidden Markov models. *IEEE ASSP Magazine,* Janvier 1986
- **CAPPÉ, MOULINES ; RYDEN DE SPRINGER.** Inference in Hidden Markov Models. *Springer series in statisctics*